# EUROPÄISCHE PATENTSCHRIFT

(11) **EP 0 317 743 B1**
(45) Veröffentlichungstag und Bekanntmachung des Hinweises auf die Patenterteilung: **15.12.1993**
(21) Anmeldenummer: 88116317.4
(22) Anmeldetag: 03.10.1988
(51) Int. Cl.: C07C 269/02, C07C 273/00

(54) **Verfahren zur Herstellung von monoblockierten (cyclo)-aliphatischen Diisocyanaten**
Process for the preparation of monoblocked (cyclo) aliphatic diisocyanates
Procédé de préparation de diisocyanates (cyclo) aliphatiques monobloqués

(30) Priorität: 21.11.1987 DE 3739477
(43) Veröffentlichungstag der Anmeldung: 31.05.1989
(73) Patentinhaber: HÜLS AKTIENGESELLSCHAFT, 45764 Marl (DE)
(72) Erfinder: Gras, Rainer, Dr., D-4630 Bochum 5 (DE); Wolf, Elmar, Dr., D-4350 Recklinghausen (DE); Disteldorf, Josef, Dr., D-4370 Marl (DE); Hübel, Werner, Dr., D-4350 Recklinghausen (DE); Schnurbusch, Horst Dr., D-4690 Herne 1 (DE)

(56) Entgegenhaltungen:
- EP-A- 0 010 248
- DE-A- 2 020 905
- FR-A- 1 043 008
- FR-A- 1 233 866
- FR-A- 2 153 295
- US-A- 3 926 875

## Beschreibung

Die vorliegende Erfindung betrifft ein Verfahren zur Herstellung von monoblockierten (cyclo)-aliphatischen Diisocyanaten.

Eine vollständige Blockierung von Isocyanaten zum temporären Schutz ihrer NCO-Gruppen ist seit langem bekannt und wird in technischem Maßstab durchgeführt, wie z. B. bei der Herstellung von 1 K-PUR-Pulver oder auch lösungsmittelhaltiger 1 K-Einbrennlacke. Aber auch die Monoblockierung von Diisocyanaten, d. h., daß pro Mol Diisocyanat ein Mol monofunktionelles Blockierungsmittel zur Reaktion kommt, gehört zum Stand der Technik. Man geht bei der Herstellung dieser monoblockierten Diisocyanate so vor, daß man zu dem Diisocyanat, das gegebenenfalls in einem inerten Lösungsmittel gelöst ist, das Blockierungsmittel portionsweise bei Raumtemperatur bis 120 °C zudosiert und so lange weiter erhitzt, bis der berechnete NCO-Gehalt erreicht ist. So wird z. B. gemäß DE-OS 31 43 060 ein mit epsilon-Caprolactam monoblockiertes Isophorondiisocyanat durch Reaktion von 1 Mol Isophorondiisocyanat (IPDI) und 1 Mol epsilon-Caprolactam bei 120 °C hergestellt.

Ein so hergestelltes, mit epsilon-Caprolactam monoblockiertes IPDI enthält aber noch ca. 16 % nicht umgesetztes IPDI. Dies ist auch verständlich, da bei einer äquimolaren Umsetzung von IPDI und epsilon-Caprolactam die Reaktion nicht so gesteuert werden kann, daß 1 Mol IPDI jeweils nur mit einem Mol epsilon-Caprolactam reagiert. In dem Maße, wie sich diblockiertes IPDI bildet, bleibt freies IPDI zurück, d. h., daß das Reaktionsprodukt aus 1 Mol IPDI und 1 Mol epsilon-Caprolactam - und dies gilt prinzipiell für alle Diisocyanate und deren Umsetzung mit monofunktionellen Blockierungsmitteln - stets ein Reaktionsgemisch aus IPDI mit epsilon-Caprolactam monoblockiertem IPDI und mit epsilon-Caprolactam diblockiertem IPDI ist.

Es war daher Aufgabe der Erfindung, einen Weg zur Herstellung eines monoblockierten Diisocyanats mit nur geringem Gehalt an freiem Diisocyanat zu finden, da dies für verschiedene Anwendungen wünschenswert ist.

Gegenstand der Erfindung ist daher ein Verfahren zur Herstellung von monoblockierten (cyclo)-aliphatischen Diisocyanaten mit einem Gehalt an freiem Diisocyanat unter 6,5 Gew.-% bis unter 1 Gew.-%, welches dadurch gekennzeichnet ist, daß man in einer 1. Stufe zu 5 bis 20 Mol Diisocyanat 1 Mol Blockierungsmittel bei Raumtemperatur bis 120 °C hinzufügt und nach dieser Reaktion in einer 2. Stufe das nicht umgesetzte Diisocyanat durch eine Dünnschichtdestillation bei 90 bis 140 °C unter Unterdruck entfernt.

Es kommen alle Diisocyanate für das erfindungsgemäße Verfahren infrage, die sich bei einer Temperatur destillieren lassen, die niedriger ist als die Deblockierungstemperatur des monoblockierten Diisocyanats. Als besonders geeignet haben sich folgende Diisocyanate erwiesen: Hexamethylendiisocyanat (HDI), 2-Methyl-1.5-diisocyanatopentan (DI51), 2-Ethyl-1.4-diisocyanatobutan, 1.5-Diisocyanatohexan, 1.10-Diisocyanatodecan, 1.9-Diisocyanato-5-methylnonan, Dodecamethylendiisocyanat, Isophorondiisocyanat (IPDI), Hexahydroxylylendiisocyanat-1.4 bzw. -1.3.

Als Blockierungsmittel werden z. B. epsilon-Caprolactam, Methylethylketoxim, oder Diisobutylketoxim eingesetzt.

Der Gehalt der erfindungsgemäß hergestellten Verbindungen an freiem NCO beträgt - je nach Diisocyanat - 11 bis 18 Gew.-%, an Gesamt-NCO 23 bis 35 Gew.-%. Die Viskosität der erfindungsgemäßen Verbindungen bei Raumtemperatur variiert in einem weiten Bereich von 100 bis 10⁶ mPas.

Das Molverhältnis von Diisocyanat zu Blockierungmsittel hängt davon ab, welcher Gehalt an diblockiertem Diisocyanat zugelassen werden kann. Je höher der Überschuß an Diisocyanat, desto niedriger ist der Gehalt an diblockiertem Diisocyanat. Bei der Umsetzung von überschüssigem Diisocyanat und Blockierungsmittel geht man so vor, daß man zu 5 bis 20 Molen Diisocyanat bei Raumtemperatur bis 120 °C 1 Mol Blockierungsmittel zudosiert und so lange weiter erhitzt, bis 1 NCO-Äquivalent verbraucht ist. Der Reaktionsverlauf wird durch Bestimmung des NCO-Gehaltes verfolgt. Ist der erwartete NCO-Gehalt erreicht, wird das Reaktionsprodukt in einer 2. Stufe vom nicht umgesetzten Diisocyanat durch eine Dünnschichtdestillation bei ca. 0,1 bis 0,2 mbar entfernt. Die Destillationstemperatur richtet sich nach dem Siedepunkt des abzudestillierenden Diisocyanats und liegt in einem Bereich von 90 bis 140 °C; für Hexamethylendiisocyanat liegt sie z. B. bei 90 °C, für IPDI bei 130 °C. Für die Abtrennung des Diisocyanats im technischen Maßstab hat es sich als zweckmäßig erwiesen, dies in zwei Schritten zu tun, d. h. in zwei hintereinandergeschalteten Dünnschichtverdampfern, wobei im 1. Dünnschichtverdampfer die Hauptmenge (ca. 80 %) und im 2. Dünnschichtverdampfer dann die restliche Menge abgetrennt wird.

Die erfindungsgemäß hergestellten Verbindungen sind in hervorragender Weise zur Herstellung von Kataphoreseharzen geeignet.

### Beispiele 1 bis 9

### Allgemeine Herstellungsvorschrift

Zu 5 bis 20 Molen Diisocyanat wird bei 70 bis 80 °C unter intensiver Rührung 1 Mol Blockierungsmittel portionsweise zugegeben. Nach beendeter Blockierungsmittelzugabe wird das Reaktionsgemisch noch ca. 1 h bei 100 °C erhitzt und anschließend das nicht umgesetzte Diisocyanat mittels Dünnschichtdestillation bei 90 bis 140 °C und 0,133 mbar entfernt. Die vom Rückstand (Reaktionsprodukt) ermittelten chemischen und physikalischen Kenndaten sind in der folgenden Tabelle zusammengefaßt.

| Beispiel | Ausgangsstoffe | | Reaktionsprodukt | | | Viskosität in mPas bei | | |
|---|---|---|---|---|---|---|---|---|
| | Diisocyanat | Blockier. mittel | NCO-frei % | NCO ges. % | Monomer % | 25 °C | 40 °C | 50 °C |
| 1 | IPDI | epsilon-Caprol. | 11,8 | 24,6 | 1,0 | 125·10⁴ | 63,5#CDT#10³ | 13,7·10³ |
| 2 | " | " | 11,5 | 24,5 | 2,2 | 114,5·10⁴ | 61,2·10³ | 12,5·10³ |
| 3 | " | " | 11,6 | 24,5 | 3,5 | 112·10⁴ | 59,5·10³ | 11,5·10³ |
| 4 | " | " | 11,8 | 24,5 | 4,6 | 66·10⁴ | 34·10³ | 8,5·10³ |
| 5 | " | MEK*-oxim | 12,8 | 26,5 | 2,8 | 57,1·10⁴ | 27,5·10³ | 5,5·10³ |
| 6 | HDI | epsilon-Caprol. | 15,3 | 29,4 | 0,5 | 100 | 50 | 30 |
| 7 | " | MEK*-oxim | 15,8 | 32,4 | 2,5 | 70 | 50 | 30 |
| 8 | DI51 | epsilon-Caprol. | 14,8 | 29,3 | 0,7 | 160 | 60 | 45 |
| 9 | " | MEK*-oxim | 15,7 | 31,5 | 0,8 | 190 | 80 | 60 |

| | | | | | | | | |
|---|---|---|---|---|---|---|---|---|
| * MEK-oxim = Methylethylketoxim | | | | | | | | |

## Patentansprüche

1. Verfahren zur Herstellung von monoblockierten (cyclo)-aliphatischen Diisocyanaten mit einem Gehalt an freiem Diisocyanat unter 6,5 Gew.-% bis unter 1 Gew.-%,
dadurch gekennzeichnet,
daß man in einer 1. Stufe zu 5 bis 20 Mol Diisocyanat 1 Mol Blokkierungsmittel bei Raumtemperatur bis 120 °C hinzufügt und nach dieser Reaktion in einer 2. Stufe das nicht umgesetzte Diisocyanat durch eine Dünnschichtdestillation bei 90 bis 140 °C unter Unterdruck entfernt.

2. Verfahren nach Anspruch 1,
dadurch gekennzeichnet,
daß der Druck 0,1 bis 0,2 mbar beträgt.

## Claims

1. A process for the manufacture of mono-blocked (cyclo)aliphatic diisocyanates having a free diisocyanate content of less than 6.5 % by weight to less than 1% by weight, characterized in that, in a first stage, 1 Mol of blocking agent is added, at room temperature to up to 120°C, to 5 to 20 Mol diisocyanate and after this reaction, in a second stage, the unreacted diisocyanate is removed by thin-film distillation at 90 to 140°C under reduced pressure.

2. A process according to claim 1, characterized in that the pressure is 0.1 to 0.2 mbar.

## Revendications

1. Procédé d'obtention de di-isocyanates (cyclo)aliphatiques monobloqués ayant une teneur an di-isocyanate libre en dessous de 6,5 % en poids à endessous de 1 % en poids, caractérisé on ce que l'on ajoute dans une première étape comprenant de 5 à 20 mol de di-isocyanate, 1 mol d'agent de blocage à une température allant de la température ambiante à 120°C et qu'après cette réaction dans une deuxième étape, on élimine le di-isocyanate qui n'a pas réagi par distillation en couche mince à une température allant de 90 à 140°C sous pression réduite.

2. Procédé selon la revendication 1, caractérisé en ce que la pression s'élève à 0,1 - 0,2 mbar.
